# EUROPÄISCHE PATENTSCHRIFT

(11) **EP 2 892 600 B1**
(45) Veröffentlichungstag und Bekanntmachung des Hinweises auf die Patenterteilung: **24.04.2019**
(21) Anmeldenummer: 13753326.1
(22) Anmeldetag: 28.08.2013
(51) Int. Cl.: A61M 25/00, A61M 3/02

(54) **TUNNELER MIT ROHR UND OLIVE**
TUNNELLING DEVICE WITH TUBE AND OLIVE
OUTIL DE TUNNELLISATION MUNI D'UN TUBE ET D'UNE STRUCTURE EN FORME D'OLIVE

(30) Priorität: 06.09.2012 DE 102012215847
(43) Veröffentlichungstag der Anmeldung: 15.07.2015
(73) Patentinhaber: B. Braun Melsungen AG, 34212 Melsungen (DE)
(72) Erfinder: WIEGEL, Heinz, 36211 Alheim (DE)
(74) Vertreter: Patentanwälte Ruff, Wilhelm, Beier, Dauster & Partner mbB
(86) Internationale Anmeldenummer: PCT/EP2013/067844
(87) Internationale Veröffentlichungsnummer: WO 2014/037267

(56) Entgegenhaltungen:
- EP-A1- 2 027 882
- WO-A1-2006/044670
- US-A1- 2012 101 433

## Beschreibung

Die Erfindung betrifft eine medizinische Tunnelungsvorrichtung zum Einführen eines Katheters in einen Patienten und zum Positionieren des Katheters in dem Patienten.

Bekannte Tunnelungsvorrichtungen weisen ein langgestrecktes, hohles Rohr auf, das ein distales und ein proximales offenes Ende aufweist. In US 2002/0052576 A1 wird eine Tunnelungsvorrichtung beschrieben, die eine Führungsnadel zum Durchstechen der Haut eines Patienten aufweist. Nachdem die Führungsnadel entfernt ist, verbleibt ein Einführkatheter zu einem Teil unterhalb der Haut des Patienten. Ein Infusionskatheter wird durch den Einführkatheter geschoben und in den zu versorgenden Wundbereich vorgeschoben. Nachdem die Positionierung des Infusionskatheters erfolgt ist, wird der Einführkatheter entfernt und eine medizinische Lösung, typischerweise ein Anästhetikum, durch den Infusionskatheter dem Wundbereich zugeführt.

Bei einer weiteren bekannten Tunnelungsvorrichtung wird ein außenliegender Splittkatheter verwendet. Der Tunneler weist zum Legen eines Schmerzkatheters einen Stahldraht auf, der beim Einführen in den Patienten Verletzungen des Gewebes verursachen kann. Nach dem Platzieren des Tunnelers muss dieser aus dem Splittkatheter herausgezogen werden, bevor anschließend der Schmerzkatheter durch den Splittkatheter vorgeschoben werden kann. Hierbei besteht eine Gefahr darin, dass der Splittkatheter beim Zurückziehen des Tunnelers die vorgesehene Position verliert. Eine weitere, bekannte Tunnelungsvorrichtung wird mit der Bezeichnung "ON-Q® Tunneler" angeboten. Diese Tunnelungsvorrichtung weist ein Einführungsrohr mit einem stumpfen, distalen Ende auf, um Beschädigungen des Gewebes eines Patienten beim Einführen der Tunnelungsvorrichtung zu reduzieren. Auch ein stumpfes Ende eines Einführungsrohres kann dennoch Verletzungen des Gewebes verursachen. Die WO2006/044670 A1 offenbart eine medizinische Tunnelungsvorrichtung gemäß dem Oberbegriff des Anspruchs 1.

Der Erfindung liegt die Aufgabe zugrunde, eine verbesserte medizinische Tunnelungsvorrichtung mit reduzierter Gefahr von Gewebeverletzungen zu schaffen.

Die erfindungsgemäße Tunnelungsvorrichtung ist definiert durch die Merkmale von Anspruch 1.

Demnach ist das distale Ende des Rohres (Einführkatheter) mit einer rundlichen, rotationssymmetrischen Verdickung versehen, deren Außendurchmesser größer ist als der Außendurchmesser des Rohres. Der Außendurchmesser der Verdickung kann beispielsweise mindestens 1,5-mal so groß und vorzugsweise etwa doppelt so groß sein wie der Außendurchmesser des Rohres. Durch die rotationssymmetrische rundliche Form ist das Risiko von Beschädigungen menschlichen Gewebes beim Einführen des Tunnelers reduziert. Beim Vorschieben des Rohres in das Gewebe wird dieses von der Verdickung in jeder Rotationslage und in jedem Einführungswinkel zur Seite geschoben, ohne dabei übermäßig zerstört zu werden.

Vorzugsweise weist die Verdickung eine olivenförmige Form auf, bei der die Verdickung in einem parallel zur Mittellängsachse des Rohres angeordneten Querschnitt weitgehend oval ausgebildet ist. Bei einer derartigen Form wird das Gewebe beim Vorschieben des Rohres besonders schonend beiseite geschoben und kann sich beim Entfernen des Rohres in seine ursprüngliche Lage zurückbewegen, ohne dabei verletzt zu werden.

An einer distalen Stirnseite der Verdickung ist die distale Öffnung des Rohres ausgebildet. Durch das Rohrlumen ist, gemäß der Erfindung, bei flexiblem Rohr ein Führungsdraht vorgeschoben werden, der mit einem abgerundeten distalen Ende versehen ist. In der Ausgangslage vor Einführen des Rohres in einen Patienten ragt das abgerundete Ende des Führungsdrahtes aus der distalen Öffnung des Rohres derart hervor, dass das abgerundete Ende des Führungsdrahtes die Rundung des distalen Endes der Verdickung vervollständigt. Dadurch wird eine homogene, stumpfe und abgerundete Form geschaffen, die beim Vorschieben der Tunnelungsvorrichtung die Gefahr von Verletzungen reduziert.

An dem dem distalen Ende des Rohres gegenüberliegenden proximalen Ende kann die medizinische Tunnelungsvorrichtung vorzugsweise ein das Rohr umgebendes Griffelement aufweisen, das beim Einführen und Positionieren der Tunnelungsvorrichtung von dem Bediener ergriffen und gehalten werden kann. Beim Einführen der Tunnelungsvorrichtung befindet sich der Führungsdraht in einer Ausgangsposition, in der das distale, abgerundete Ende des Führungsdrahtes über das distale Ende der Verdickung und des Rohres hinausragt. Der Führungsdraht stabilisiert das flexible Rohr. Durch geeignete Hebel-, Dreh- und Vorschubbewegungen wird das Rohr in den Patienten eingeführt und das distale Ende des Rohres an der erforderlichen Position platziert. Je nach Notwendigkeit kann der Anwender das flexible Rohr den anatomischen Anforderungen gemäß (vor)biegen. Anschließend wird der Führungsdraht entfernt und durch das innere Lumen des Rohres der Katheter vorgeschoben. Zuletzt wird dann die Tunnelungsvorrichtung über den Katheter zurück aus dem Patienten herausgezogen, so dass der Katheter in dem Stichkanal verbleibt. Ein splittbarer Katheter über der Tunnelungsvorrichtung wird nicht benötigt.

Im Folgenden wird anhand der Figuren ein Ausführungsbeispiel der Erfindung näher erläutert. Es zeigen:
- Fig. 1: eine perspektivische Ansicht und
- Fig. 2: einen Schnitt durch die Mittellängsachse des distalen Endes.

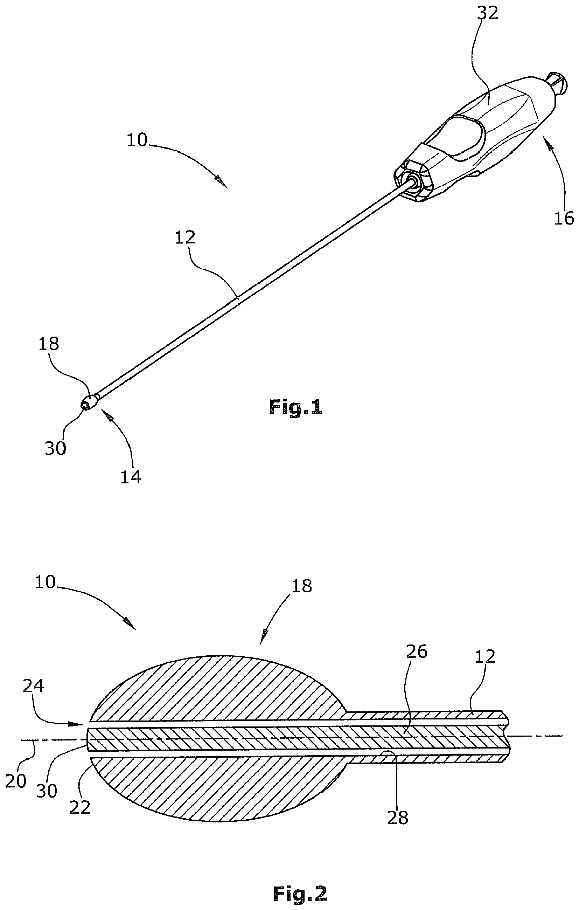

Die Tunnelungsvorrichtung 10 ist mit einem langgestreckten, hohlen Rohr 12 versehen, das ein distales Ende 14 und ein dem distalen Ende 14 gegenüberliegendes proximales Ende 16 aufweist. Am distalen Ende 14 ist das Rohr 12 mit einer rotationssymmetrischen, rundlichen Verdickung 18 versehen. In dem in Fig. 2 dargestellten Schnitt durch die Mittellängsachse 20 des Rohres 12 ist zu erkennen, dass die Verdickung 18 einen annähernd ovalen Querschnitt und dadurch eine olivenförmige Gestalt aufweist.

An der distalen Stirnseite 22 der Verdickung 18 ist die distale Öffnung 24 des Rohres 12 ausgebildet, durch die der Führungsdraht 26 und der Katheter vorgeschoben werden. Die distale Öffnung 24 bildet das Ende 14 des Lumens 28, das entlang der Mittellängsachse 20 und konzentrisch um diese herum durch das Rohr 12 und durch die Verdickung 18 hindurch verläuft. Das Lumen 28 verläuft vollständig durch das Rohr 12 von dessen distalem Ende 14 bis zu dessen proximalem Ende 16 hindurch.

Der Führungsdraht 26 ist in dem Lumen 28 enthalten. Dabei zeigen die Figuren den Ausgangszustand der Tunnelungsvorrichtung 10 vor dem Einführen in einen Patienten. Der Führungsdraht 26 ist mit einem abgerundeten Ende 30 versehen, das etwas über die distale Stirnseite 22 der Verdickung 18 hinaus aus der Öffnung 24 hervorsteht. Die Rundung des abgerundeten Endes 30 ergänzt dadurch die Rundung der Verdickung 18 zu einer annähernd homogenen Olivenform, die beim Einführen in einen Patienten das Verletzungsrisiko reduziert.

Im Bereich des proximalen Endes 16 ist an dem Rohr 12 ein Griffelement 32 als Handgriff 32 ausgebildet. Über den Handgriff 32 können die zum Positionieren der Tunnelungsvorrichtung 10 erforderlichen Vorschub-, Dreh- und Hebelbewegungen ausgeführt werden. Über den steifen, stabilen Führungsdraht 26 werden diese Bewegungen auf das distale Ende 14 des Rohres 12 und auf die Verdickung 18 übertragen. Nach erfolgter Positionierung des distalen Rohrendes 14 wird der Führungsdraht 26 aus dem Rohr 12 entfernt und anschließend ein Katheter durch das Rohrlumen 28 vorgeschoben.

## Patentansprüche

1. Medizinische Tunnelungsvorrichtung (10) zum Einführen und Positionieren eines Katheters in einen Patienten, mit einem langgestreckten, hohlen Rohr (12), das ein distales und ein proximales offenes Ende (14, 16) aufweist, wobei das distale Ende (14) mit einer rundlichen, rotationssymmetrischen Verdickung (18) versehen ist,
**dadurch gekennzeichnet,**
**dass** das Rohr (12) flexibel mit einem in dem Rohrlumen (28) enthaltenen, aus dem Rohr (12) entfernbaren Führungsdraht (26) ausgebildet ist, der mit einem abgerundeten, distalen Ende (14) versehen ist, und
**dass** der Führungsdraht (26) derart in dem Rohr (12) angeordnet ist, dass das distale Ende (14) des Führungsdrahtes (26) aus der distalen Öffnung (24) des Rohres (12) herausragt und die Rundung des distalen Endes (14) der Verdickung (18) vervollständigt.

2. Tunnelungsvorrichtung (10) nach Anspruch 1, **dadurch gekennzeichnet, dass** die Verdickung (18) in einem parallel zur Mittellängsachse (20) des Rohres (12) angeordneten Querschnitt im Wesentlichen oval ausgebildet ist, so dass die Verdickung (18) olivenförmig ist.

3. Tunnelungsvorrichtung (10) nach Anspruch 1 oder 2, **dadurch gekennzeichnet, dass** die Verdickung (18) an ihrer distalen Stirnseite (22) die distale Öffnung (24) des Rohres (12) aufweist.

4. Tunnelungsvorrichtung (10) nach einem der vorhergehenden Ansprüche, **dadurch gekennzeichnet, dass** im Bereich des proximalen Endes (16) des Rohres (12) ein das Rohr (12) umgebendes Griffelement (32) zum Halten und Führen des Rohres (12) vorgesehen ist.

## Claims

1. Medical tunneling device (10) for inserting and positioning a catheter into a patient, comprising an elongate, hollow tube (12) which has a distal end and a proximal end (14, 16), wherein the distal end (14) is provided with a rounded, rotationally symmetric thickening (18),
**characterized in that**
the tube (12) is flexible with a guide wire (26) that is removable from the tube (12) and included in the lumen (28) of the tube, which guide wire is provided with a rounded distal end (14), and
**in that** the guide wire (26) is arranged within the tube (12) in such a manner that the distal end (14) of the guide wire (26) protrudes from the distal opening (24) of the tube (12) and completes the rounding of the distal end (14) of the thickening (18).

2. Tunneling device (10) according to claim 1, **characterized in that** the thickening (18), in a cross section disposed in parallel to the central longitudinal axis (20) of the tube (12), has an essentially oval shape such that the thickening (18) has an olive shape.

3. Tunneling device (10) according to claim 1 or 2, **characterized in that** the thickening (18) includes the distal opening (24) of the tube (12) on the distal face end side (22) thereof.

4. Tunneling device (10) according to any of the preceding claims, **characterized in that** in the vicinity of the proximal end (16) of the tube (12) a handle member (32) enclosing the tube (12) is provided for holding and guiding the tube (12).

## Revendications

1. Dispositif de tunnelisation médical (10) permettant d'introduire et de positionner un cathéter chez un patient, muni d'un tube creux allongé (12), qui présente une extrémité distale et une extrémité proximale (14, 16), dans lequel l'extrémité distale (14) est munie d'une surépaisseur arrondie à symétrie de rotation (18),
**caractérisé en ce que** le tube (12) est flexible avec un fil de guidage (26) contenu dans la lumière du tube (28) et amovible hors du tube (12), lequel fil de guidage es pourvu d'une extrémité distale (14) arrondie, et **en ce que** le fil de guidage (26) est disposé dans le tube (12), de telle manière que l'extrémité distale (14) du fil de guidage (26) sorte de l'ouverture distale (24) du tube (12) et complète l'arrondi de l'extrémité distale (14) de la surépaisseur (18).

2. Dispositif de tunnelisation (10) selon la revendication 1, **caractérisé en ce que** la surépaisseur (18) est de forme essentiellement ovale dans une section transversale disposée parallèlement à l'axe longitudinal central (20) du tube (12), de telle manière que la surépaisseur (18) soit en forme d'olive.

3. Dispositif de tunnelisation (10) selon une revendication 1 ou 2, **caractérisé en ce que** la surépaisseur (18) présente sur son côté frontal distal (22) l'ouverture distale (24) du tube (12).

4. Dispositif de tunnelisation (10) selon l'une quelconque des revendications précédentes, **caractérisé en ce qu'**il est prévu dans la région de l'extrémité proximale (16) du tube (12) un élément de prise (32) entourant le tube (12) pour le maintien et le guidage du tube (12).
